# EUROPÄISCHE PATENTSCHRIFT

(11) **EP 3 020 442 B1**
(45) Veröffentlichungstag und Bekanntmachung des Hinweises auf die Patenterteilung: **22.01.2020**
(21) Anmeldenummer: 15194182.0
(22) Anmeldetag: 11.11.2015
(51) Int. Cl.: A61M 25/09, A61M 25/01, A61B 90/00

(54) **DRAHTFÖRMIGES MEDIZINISCHES INSTRUMENT SOWIE VERFAHREN ZU SEINER HERSTELLUNG**
WIRE-SHAPED MEDICAL INSTRUMENT AND METHOD FOR PRODUCING THE SAME
INSTRUMENT MEDICAL FILIFORME ET SON PROCEDE DE FABRICATION

(30) Priorität: 12.11.2014 DE 102014116566
(43) Veröffentlichungstag der Anmeldung: 18.05.2016
(73) Patentinhaber: Nano4Imaging GmbH, 40225 Düsseldorf (DE)
(72) Erfinder: Manegold, Christoph R., 40549 Düsseldorf (DE); Borm, Paul J. A., 6231 CV Meerssen (NL); Cremers, Jozef G. O., 6411 LP Heerlen (NL)
(74) Vertreter: Naeven, Ralf

(56) Entgegenhaltungen:
- DE-A1-102007 016 674
- DE-A1-102011 081 445
- DE-A1-102012 214 785
- US-A1- 2014 121 648

## Beschreibung

Die Erfindung betrifft ein drahtförmiges medizinisches Instrument gemäß dem Oberbegriff des Anspruchs 1 sowie ein Verfahren zu seiner Herstellung.

Die DE 102007016674 A1 offenbart ein medizinisches Instrument der eingangs genannten Art mit einem gelförmigen oder flüssigen Markierungsmittel in einem zentralen Markierungslumen. Das als Katheter oder Führungsdraht ausgebildete medizinische Instrument kann in der Magnetresonanztomographie (MRT) eingesetzt werden, wobei die Markierungsmittel dazu dienen, das Signal des Instruments in der MRT-Bildgebung zu verstärken oder zu schwächen und damit eine verbesserte Sichtbarkeit zu erreichen.

Die DE 102011081445 A1 offenbart ein medizinisches Instrument der eingangs genannten Art in Form eines Führungsdrahts sowie ein Verfahren zu seiner Herstellung. Es ist offenbart, im Kern des Führungsdrahtes einen Drahtfaden vorzusehen, welcher aus mindestens einem matrixfreien Faserverbund gebildet ist, wobei der Faserverbund aus mehreren seil- oder litzenbildend zusammengefassten nichtmetallischen Fasern gebildet ist. In den Zwischenräumen zwischen den Fasern ist ein MR-Marker eingebracht. Zur Herstellung des Führungsdrahtes ist es offenbart, den MR-Marker in Form von Partikeln in einer niederviskosen Lösung zu dispergieren, mit welcher der aus den einzelnen Fasern vorgefertigte Faserverbund getränkt wird, so dass die Lösung in die Zwischenräume eindringen kann. Die Lösung kann dann abgedampft werden oder auch in den Zwischenräumen verbleiben. Der Drahtkern ist von einer Ummantelung umgeben, die am vorderen Ende biegeweich und ansonsten biegesteif sein kann.

Die EP 1 603 622 B1 offenbart ebenfalls ein medizinisches Instrument der eingangs genannten Art mit einem in Markierungslumina angeordneten Markierungsmittel, welches die T1 Relaxationszeit bei der Magnetresonanz-Bildgebung verringert und einen Seltene-Erden-Komplex, wie z.B. Gd-DTPA, enthalten kann. Das Markierungsmittel kann auch Wasser oder Glycerin enthalten und mithin flüssig sein. Die Markierungslumina sind z.B. aus einem Kunststoff gebildete Hohlkörper, die durch Extrusion, Ziehverfahren oder Gießen hergestellt werden und sich über die gesamte Länge des medizinischen Instruments erstrecken können. Alternativ sind die Hohlkörper als kurze Hohlfasern offenbart, die auch vor einer Extrusion mit dem Kunststoff der Matrix des medizinischen Instruments vermischt werden können. Die Hohlkörper können durch Injektion oder mittels Tauchens mit dem Markierungsmittel gefüllt werden.

Aus der DE 695 32 786 T2 ist ein MR-sichtbarer Katheter bekannt mit einem aus einem Plastikmaterial extrudierten rohrförmigen Körper mit einem proximalen und einem distalen Ende und mit zumindest einem zur Anwendung des Katheters freibleibenden Lumen. Für die MR-Sichtbarkeit dient ein in Plastikmaterial eingefügter Füllstoff aus einem paramagnetischen Material, wobei das das paramagnetische Material enthaltende Plastikmaterial eine Zwischenschicht von zumindest drei koaxialen Schichten des rohrförmigen Körpers bildet.

Die US 5,782,764 A offenbart ein medizinisches Instrument, insbesondere eine Biopsienadel mit einem Körper aus z.B. einem Kohlenstofffasern oder Glasfasern aufweisenden Verbundmaterial. Zur Sichtbarmachung bei der Magnetresonanz-Bildgebung dient ein vorzugsweise flüssiges Markierungsmittel, welches z.B. einen organischen Chelatkomplex eines paramagnetischen Ions, z.B. Gd-DTPA, enthalten kann. Das Markierungsmittel kann in einem auswechselbaren Markierungsobjekt im Inneren des Instruments angeordnet oder alternativ in Kammern oder Kapillaren vorgesehen werden, welche im Körper des Instruments integriert sind oder am Umfang entfernbar angeordnet werden.

Die US 5,154,179 A offenbart einen Katheter aus Kunststoff, welcher zur Sichtbarmachung in der Magnetresonanz-Bildgebung ferromagnetische Partikel im Kunststoff des Katheterkörpers aufweist. Die ferromagnetischen Partikel können dabei über die gesamte Länge oder in bestimmten Bereichen des Katheters, z.B. in voneinander beabstandeten Bändern, vorgesehen werden, welche in Umfangsrichtung verlaufen. Hierfür können die ferromagnetischen Partikel vor oder während der Extrusion des Katheterkörpers dem Kunststoff beigemischt werden. Des Weiteren ist es offenbart, die ferromagnetischen Partikel in sich in Längsrichtung des Katheters erstreckenden Streifen vorzusehen, indem die Partikel während der Extrusion nur in bestimmten Umfangsbereichen zugeführt werden. Es ist auch offenbart, ein flüssiges oder gelförmiges Markierungsmittel vorzusehen und dieses in ein Seitenlumen einzubringen, welches sich in Längsrichtung des Katheters erstreckt. Das Seitenlumen kann über eine gesonderte Eintrittsöffnung vor oder während einer bestimmungsgemäßen Benutzung des Katheters eingefüllt werden. In einer anderen Ausführungsform ist es offenbart, das Markierungsmittel in ein zentrales Lumen einzubringen, welches ansonsten zum Entleeren oder Aufblasen eines Ballons genutzt wird und vom Hauptlumen des Katheters umgeben ist. Es ist offenbart, als Markierungsmittel ein paramagnetisches Agens vorzusehen, welche T1- und T2-Charakteristika aufweisen, welche sich von denen des umgebenden Gewebes unterscheiden. Das Agens kann in wässrigen Lösungen oder Suspensionen enthalten sein. Beispielhaft sind als paramagnetische Substanzen Ionen der Metalle Gadolinium, Chrom, Nickel, Kupfer, Eisen und Mangan genannt. Anders als das medizinische Instrument der eingangs genannten Art weist der in US 5,154,179 A offenbarte Katheter keinen Drahtkern aus einem Faserverbundmaterial auf.

Aus der DE 10 2006 020 402 B3 ist ein Führungsdraht für einen Mikrokatheter zum Einsatz im Gehirn eines Patienten bekannt, der magnetische Nanopartikel in Form eines Ferrofluids oder in Pulverform aufweist und mit Hilfe von Magnetfeldern navigiert werden kann. Der offenbarte Führungsdraht besteht aus einem weichen biegsamen Kunststoff.

Aus der US 2014/0121648 A1 ist ein MRT-geeigneter Führungsdraht bekannt, bei dem das vordere Ende einen Sensor aufweist, während der mit Fasern verstärkte Schaft des Führungsdrahtes ein oder mehrere mit dem Sensor verbundene Kabel aufweist, insbesondere aus optischen Glasfasern. Der Schaft ist an seinem vorderen Ende kegelförmig verjüngt Ein gelförmiges oder flüssiges Markierungsmittel ist nicht offenbart. Zur Herstellung des Schafts aus Verbundmaterial ist ein Pultrusionsverfahren vorgeschlagen, wobei während des Pultrusionsprozesses das Kabel in den Schaft eingearbeitet werden kann.

Aus der DE 102012214785 A1 ist ein medizinischer Führungsdraht bekannt, der einen Drahtkern aus einem MR-unsichtbaren Material umfasst, der von einer Ummantelung umgeben ist. Die Ummantelung weist mindestens zwei Vollmaterial- und/oder Faser-Schichtlagen auf, die durch unterschiedliche, MR-unsichtbare Kunststoffmaterialien gebildet sind. Wenigstens ein MR-Markerelement ist in den mehrlagigen Schichtaufbau der Ummantelung integriert oder von diesem umgeben. Es sind partikel- oder linienförmige Markerelemente offenbart.

Es ist nun Aufgabe der vorliegenden Erfindung, ein drahtförmiges medizinisches Instrument der eingangs genannten Art sowie Verfahren zu dessen Herstellung zur Verfügung zu stellen, die eine gegenüber dem Stand der Technik veränderte Struktur bzw. Vorteile bei der Herstellung und der Sichtbarkeit bei der Magnetresonanz-Bildgebung aufweisen

Diese Aufgabe wird in Bezug auf ein medizinisches Instrument der eingangs genannten Art mit den kennzeichnenden Merkmalen des Anspruchs 1 gelöst.

Demnach werden folgende Möglichkeiten der Einkapselung eines flüssigen oder gelförmigen, für die Magnetresonanztomografie (MRT) geeigneten Markierungsmittels im Drahtkern eines drahtförmigen medizinisches Instruments erstmals vorgeschlagen, wobei diese Möglichkeiten alternativ oder kumulativ eingesetzt werden können.

Um den Drahtkern herum kann ein Markierungslumen mit einem ringförmigen Querschnitt gebildet werden, welches innen vom Drahtkern und außen von einem ersten Mantelelement begrenzt wird. Des Weiteren ist es möglich, um das erste Mantelelement herum ein zweites Mantelelement vorzusehen, wobei zwischen dem zweiten Mantelelement und dem ersten Mantelelement ein ringförmiges Markierungslumen vorgesehen wird. Dieses Prinzip kann grundsätzlich durch weitere Mantelelemente fortgesetzt werden. Auf diese Weise ist es möglich, das medizinische Instrument mit mehreren in radialer Richtung beabstandeten Markierungslumina zu versehen und damit einen besonders wirkungsvollen Kontrast für die MR-Bildgebung zu erreichen. Des Weiteren wäre es auch denkbar, unterschiedliche Markierungslumina mit unterschiedlichen Markierungsmitteln zu versehen, die für unterschiedliche Bildgebungsverfahren optimiert sind, z.B. neben MR auch MPI (Magnetic Particle Imaging) und Verfahren unter Einsatz von Röntgenstrahlung.

Zusätzlich kann das Markierungsmittel in mindestens einem Markierungslumen eingekapselt sein, welches durch die Schlauchform des Drahtkerns gebildet ist. Wird der Drahtkern in seiner gesamten oder nahezu gesamten Länge zur Bereitstellung des Markierungslumens genutzt, kann sein gesamter Verlauf während des Einsatzes im MR-Verfahren sichtbar bleiben. Es ist allerdings auch denkbar, den Drahtkern auf einen Abschnitt beschränkt mit einem Markierungslumen oder mit in axialer Richtung mit Abstand zueinander angeordneten Markierungslumina zu versehen. Vorzugsweise ist das Markierungslumen oder ist zumindest eines der Markierungslumina des Drahtkerns dauerhaft geschlossen.

Es kann vorteilhaft sein, das erfindungsgemäße medizinische Instrument so auszubilden, dass das Markierungslumen oder mindestens eines der Markierungslumina des Drahtkerns von einem schlauchförmigen zentralen Lumenelement aus Kunststoff begrenzt wird. Das Lumenelement kann z.B. aus einem Polymer, z.B. Polyurethan (PU), einem thermoplastischen Elastomer (TPE), wie z.B. Pebax ®, Polyetheretherketon (PEEK), Polytetrafluorethylen (PTFE) oder Perfluorethylenpropylen (FEP) hergestellt sein.

Es kann auch vorteilhaft sein, das erfindungsgemäße medizinische Instrument so auszubilden, dass der Drahtkern, der ohne oder mit Markierungslumen oder Markierungslumina ausgebildet sein kann, mindestens zwei Schichten mit jeweils mindestens einer Wicklung einer oder mehrerer Verstärkungsfasern umfasst, wobei mindestens zwei der Schichten unterschiedliche Wicklungsrichtungen aufweisen. Bei den Verstärkungsfasern kann es sich z.B. um Aramidfasern handeln. Mit unterschiedlichen Wicklungsrichtungen lassen sich mechanischen Eigenschaften des medizinischen Instruments wirkungsvoll einstellen, insbesondere eine hohe Torsionssteifigkeit bei gleichzeitig geringer Biegesteifigkeit. So können bei Blick in axialer Richtung z.B. eine Schicht mit Wicklung im Uhrzeigersinn und die andere Schicht mit Wicklung gegen den Uhrzeigersinn bei Wicklungswinkeln gegenüber der Längsachse des Drahtkerns zwischen 0° und 90° ausgestattet sein.

Das erfindungsgemäße medizinische Instrument kann auch so ausgebildet sein, dass der Drahtkern an seinem distalen Ende einen ersten Endbereich mit einem gegenüber einem Hauptdurchmesser des Drahtkerns verringerten ersten Enddurchmesser aufweist und das erste Mantelelement den ersten Endbereich umgibt. Somit kann die Spitze des medizinischen Instruments trotz des ersten Mantelelements im Durchmesser gering gehalten werden. Dabei ist es vorteilhaft, wenn das erste Mantelelement sich lediglich über den ersten Endbereich erstreckt. Damit kann ein ringförmiges, auf den ersten Endbereich beschränktes Markierungslumen gebildet werden, so dass es möglich ist, die Markierung im Bildgebungsverfahren auf die Spitze des medizinischen Instruments zu beschränken und so die Spitze des das erfindungsgemäße medizinische Instrument hervorzuheben und in besonderer Weise sichtbar zu machen. Eine derartige Markierung der Spitze kann auch zusätzlich zum Einsatz von Markierungsmitteln im Drahtkern vorgesehen werden, so dass dieser in seiner Länge oder in Teilen sichtbar ist und die Spitze einen verstärkten Kontrast aufweist.

In einer weiteren Ausführungsform des erfindungsgemäßen medizinische Instruments kann vorgesehen werden, dass der Drahtkern einen in Richtung auf das proximale Ende sich an den ersten Endbereich anschließenden zweiten Endbereich mit einem zweiten Enddurchmesser aufweist, wobei der zweite Enddurchmesser kleiner als der Hauptdurchmesser und größer als der erste Enddurchmesser ist, und wobei das erste Mantelelement allein den ersten Endbereich und das zweite Mantelelement das erste Mantelelement und den zweiten Endbereich umgibt. Dieses grundsätzlich durch weitere Mantelelemente erweiterbare Prinzip erhöht die Variationsmöglichkeiten in der Bildgebung, z.B. durch unterschiedliche Kontrastbereiche am distalen Ende des medizinischen Instruments.

Es kann vorteilhaft sein, das erfindungsgemäße medizinische Instrument so auszubilden, dass das Markierungsmaterial ein Gel ist. Ein Gel ist ein einphasiges System aus einem kolloidalen oder polymeren Netzwerk, dessen Poren durch ein Fluid gefüllt sind. insbesondere kann es vorteilhaft sein, ein Hydrogel einzusetzen, welches als Fluid Wasser umfasst.

Das Markierungsmaterial umfasst ein für das Magnetresonanz-Bildgebungsverfahren geeignetes Agens, vorzugsweise ein solches, welches einen positiven Kontrast erzeugt, weiter vorzugsweise ein solches, welches die T1-Relaxationszeit und/oder die T2-Relaxationszeit reduziert. Hierzu gehören z.B. die Salze der Lanthaniden Gd³⁺, Ho³⁺, Dy³⁺, Eu³⁺, die Komplexe einiger Übergangsmetalle, wie Fe³⁺ (z.B. Hämin), Mn²⁺, Mn³⁺ und Co³⁺. Derartige Agenzien ermöglichen die Sichtbarkeit des medizinischen Instruments in den gängigen MRT-Verfahren ohne besondere Maßnahmen. Weitere Agenzien, auch solche, die in anderen Bildgebungsverfahren, z.B. mittels Röntgenstrahlen, positive oder negative Kontraste erzeugen, können ebenfalls vorgesehen sein.

Das Markierungsmaterial ist vorzugsweise biokompatibel und/oder biologisch abbaubar, um im Falle eines Bruches des medizinischen Instruments Schädigungen eines betroffenen Patienten durch das Markierungsmaterial zu vermeiden.

Bei einem zur Herstellung eines medizinischen Instruments nach einem der Ansprüche 1 bis 8 geeigneten Verfahren, wird das technische Problem durch die Merkmale des Anspruchs 9 gelöst.

Das Verfahren nach Anspruch 9 bezieht sich auf die Variante mit einem ersten um den Drahtkern herum angeordneten Mantelelement. Das Mantelelement kann beispielsweise in einem Extrusionsverfahren hergestellt werden. Es kann vorgesehen werden, zwischen dem ersten Endabschnitt und dem ersten Mantelelement eine Schicht aus flüssigem oder gelförmigem Markierungsmittel einzurichten. Das Markierungsmittel kann per Injektion in das Markierungslumen zwischen erstem Mantelelement und Drahtkern eingebracht werden. Es ist aber auch möglich, das flüssige oder gelförmige Markierungsmittel in das erste Mantelelement zu füllen und anschließend den Drahtkern einzuführen. Dabei gegebenenfalls austretendes Markierungsmittel kann anschließend entfernt werden. Möglicherweise verbleibende Lücken zwischen Drahtkern und dem Ende des Mantelelements können mit geeigneten Mitteln, z.B. einem Klebstoff, verschlossen werden. Das Mantelelement kann schlauchförmig und beidseitig offen sein. Das Mantelelement kann auch schlauchförmig mit einem offenen und einem distalen geschlossenen Ende sein. Nach Anbringen des Mantelelements offene Stellen können mit anderen Materialien, zum Beispiel einem Klebstoff, verschlossen werden. Diese Gestaltungsmöglichkeiten gelten auch für gegebenenfalls weitere Mantelelemente am medizinischen Instrument.

Es kann vorteilhaft sein, das erfindungsgemäße Verfahren so auszuführen, dass der Drahtkern an seinem distalen Ende in einem ersten Endabschnitt gegenüber einem Hauptdurchmesser des Drahtkernes mit einem ersten Enddurchmesser versehen und der erste Endabschnitt mit einem ersten Mantelelement umgeben wird. Die Verringerung des Durchmessers kann z.B. im Anschluss an das Pultrusionsverfahren durch Abschleifen erfolgen.

Bei dem erfindungsgemäßen medizinischen Instrument handelt es sich vorzugsweise um einen Führungsdraht oder um einen Steuerdraht, z.B. zur Kraftübertragung bei der Steuerung der Bewegung von Elementen einer Gerätschaft, z.B. einer Biopsienadel.

Die Variante nach Anspruch 15 betrifft das medizinische Instrument mit mindestens einem Markierungslumen im Drahtkern, welches z.B. nach dem Pultrusionsverfahren vorzugsweise durch Eintauchen oder durch Injektion mit dem Markierungsmittel gefüllt werden kann. Erforderlichenfalls kann nach dem Befüllen das jeweilige Markierungslumen mittels eines Klebstoffes oder anderweitig dauerhaft geschlossen werden.

Weitere Ausführungsformen des erfindungsgemäßen Verfahrens ergeben sich aus den Unteransprüchen 12 bis 14 und 16.

Beispielhafte Ausführungsformen des erfindungsgemäßen medizinischen Instruments sowie des erfindungsgemäßen Verfahrens sind im Folgenden anhand von Prinzipzeichnungen dargestellt. Der Einfachheit halber wird im Folgenden das medizinische Instrument als Führungsdraht betrachtet. Entsprechendes gilt auch für andere drahtförmige medizinische Instrumente, z.B. einem Steuerdraht.

Es zeigt schematisch:
Fig. 1: ausschnittsweise einen Führungsdraht in einer ersten Ausführungsform im seitlichen Querschnitt,
Fig. 2: ausschnittsweise einen Führungsdraht in einer zweiten Ausführungsform im seitlichen Querschnitt und
Fig. 3: eine Vorrichtung zur Herstellung eines Führungsdrahtes.

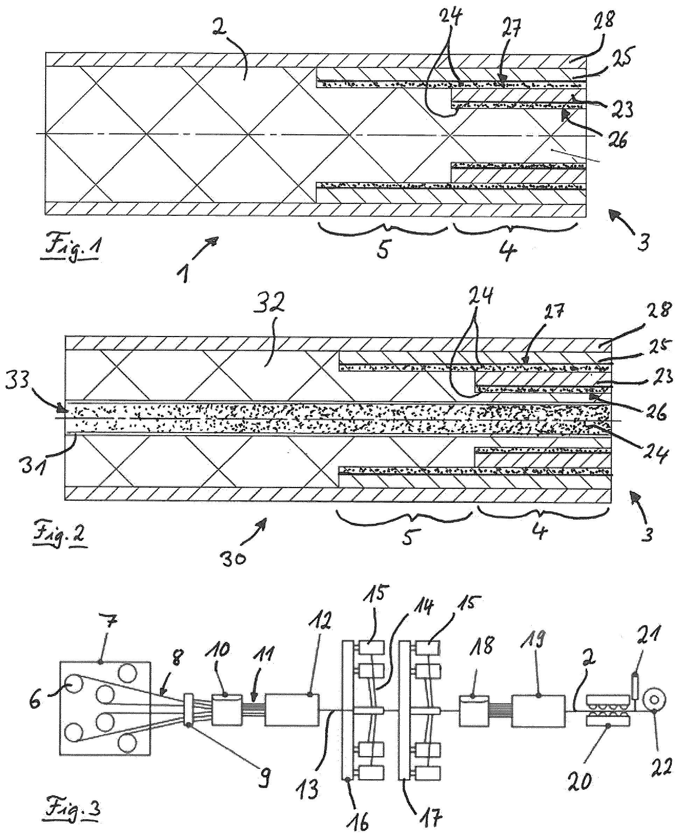

Ein in Fig. 1 ausschnittsweise und nicht maßstäblich dargestellter Führungsdraht 1 weist einen Drahtkern 2 auf, der zu seinem distalen Ende 3 hin einen ersten Endabschnitt 4 und einen zweiten Endabschnitt 5 aufweist. Der Durchmesser des zweiten Endabschnitte 5 ist gegenüber dem Hauptdurchmesser des sich in Fig. 1 links am zweiten Endabschnitt 5 anschließenden Teil des Drahtkerns 2 verringert. Der erste Endabschnitt 4 des Drahtkerns 2 weist einen gegenüber dem zweiten Endabschnitt 5 weiter verringerten Durchmesser auf. Die Durchmesserverringerung wird vorzugsweise durch Schleifen erzeugt. In der Praxis können die Übergänge zwischen den Durchmessern auch allmählich sein. Beispielhaft wird der Drahtkerndurchmesser von einem Hauptdurchmesser von z.B. 0,65 mm auf 0,35 mm im zweiten Endabschnitt 5 mm und weiter auf 0,25 mm im ersten Endabschnitt 4 verringert. Der erste Endabschnitt 4 ist beispielsweise 60mm und der zweite Endabschnitt 30 mm lang.

Anhand der schematischen Darstellung in Fig. 3 wird eine mögliche Herstellungsweise des Führungsdrahtes 1 mittels eines Pultrusionsverfahrens erläutert. Von einer eine Mehrzahl von Faserrollen 6 aufweisenden Faserquelle 7 werden erste Verstärkungsfasern 8, z.B. aus Glas und/oder Aramid, über eine Faserführung 9 einem Harzinjektor 10 zugeführt, wo die Verstärkungsfasern mit einem Harz getränkt werden. Das mit Harz getränkte Faserbündel 11 wird anschließend durch ein Formwerkzeug 12 geführt, beispielsweise einen Ziehring. Der geformte Faserverbundstrang 13 wird anschließend mit Wickelfasern 14, z.B. aus Aramid, umwickelt, die von auf Wickelscheiben 16 und 17 angeordneten Wickelfaserrollen 15 entnommen werden. Für den Wickelvorgang drehen sich die Wickelscheiben 16 und 17 gegenläufig um eine zur Ziehrichtung des Faserverbundstranges 13 koaxiale Drehachse. Der Wickelwinkel relativ zur Längsachse des Faserverbundstrangs 13 wird durch die dem Faserverbundstrang 13 aufgegebene Ziehgeschwindigkeit und die Drehgeschwindigkeit der Wickelscheiben 16 und 17 bestimmt.

Der mit den Wickelfasern 14 umwickelte Faserverbundstrang 13 wird anschließend in einem zweiten Harzinjektor 18 wiederum mit Harz getränkt und einem zweiten Formwerkzeug 19 zum Drahtkern 2 geformt. In Ziehrichtung hinter dem zweiten Formwerkzeug 19 ist eine Zieheinheit 20 dargestellt. Hinter der Zieheinheit 20 wird der Drahtkern 2 mit einer Schneidevorrichtung 21 geschnitten und anschließend in den gewünschten Längen auf einer Rolle 22 aufgerollt.

Der Drahtkern 2 wird nun am distalen Ende 3 (Fig. 1) geschleift, um die bereits erwähnten Endabschnitte 4 und 5 zu erzeugen. Der erste Endabschnitt 4 ist von einem ersten Mantelelement 23 umgeben, welches vorzugsweise aus einem Polymer z.B. PEEK, PTFE oder Pebax ® mittels Extrusion hergestellt ist. Zwischen dem Drahtkern 2 im ersten Endabschnitt 4 und dem ersten Mantelelement 23 liegt ein Markierungslumen 26 vor, welches mit einem Markierungsmittel 24 gefüllt ist. Das Markierungsmittel 24 enthält mindestens ein in den Figuren nicht sichtbares Agens, welches bei der Magnetresonanz-Bildgebung eine Verkürzung der T1 -Relaxationszeit und/oder der T2-Relaxationszeit bewirkt. Die beschrieben Struktur kann vorzugsweise hergestellt werden, indem der erste Endabschnitt 4 in das das Markierungsmittel 24 aufweisende erste Mantelelement 23 eingesteckt wird. Beim Einschieben des ersten Endabschnittes 4 des Drahtkerns 2 verbleibt zwischen dem ersten Endabschnitt 4 und dem ersten Mantelelement 23 zumindest ein Teil des Markierungsmittels 24, wodurch das erste Markierungslumen 26 entsteht. Überfließendes Markierungsmittel 24 wird entfernt.

Ein zweites Mantelelement 25 umgibt das erste Mantelelement 23 sowie den zweiten Endabschnitt 5, wobei ein zweites Markierungslumen 27 gebildet wird, welches mit z.B. demselben Markierungsmittel 24 gefüllt ist. Auch diese Struktur kann vorzugsweise durch Einstecken, nämlich des ersten Endabschnitts 4 mit dem ersten Mantelelement 23 und des zweiten Endabschnittes 5 in das das Markierungsmittel 24 aufweisende zweite Mantelelement 25, gebildet werden. Im zweiten Markierungslumen 27 kann auch ein vom Markierungsmittel 24 abweichendes Markierungsmaterial verwendet werden.

Über den Drahtkern 2 und dem zweiten Mantelelement 25 wird noch ein abschließender Außenmantel 28, z.B. aus Pebax ® gezogen, der auch BaSO₄ aufweisen kann.

Fig. 2 zeigt einen alternativen Führungsdraht 30, der sich vom Führungsdraht 1 der Fig. 1 durch einen anders gestalteten Drahtkern 32 unterscheidet. Der Drahtkern 32 weist in seinem Inneren einen Kunststoffschlauch 31 auf, der ein zentrales Markierungslumen 33 begrenzt, welches mit Markierungsmittel 24 gefüllt ist. Auf diese Weise kann der Führungsdraht 30 auf seiner ganzen Länge in der MR-Bildgebung sichtbar gemacht werden. Ansonsten stimmt der Aufbau mit dem des Führungsdrahtes gemäß Fig. 1 überein, weshalb auf die diesbezügliche Beschreibung verwiesen wird.

In Bezug auf die Herstellung des Führungsdrahtes 30 gemäß Fig. 2 kann auf die Beschreibung zu Fig. 1 und Fig. 3 verwiesen werden, mit der Maßgabe, dass der Faserführung 9 und dem ersten Harzinjektor 10 neben den Verstärkungsfasern auch der das zentrale Markierungslumen 33 begrenzende Kunststoffschlauch 31 zugeführt wird. Die Befüllung des zentralen Markierungslumens 33 erfolgt vorzugsweise mittels Injektion.

**Bezugszeichenliste**

| | | | |
|---|---|---|---|
| 1 | Führungsdraht | 31 | Kunststoffschlauch |
| 2 | Drahtkern | 32 | Drahtkern |
| 3 | distales Ende | 33 | zentrales Markierungslumen |
| 4 | erster Endabschnitt | | |
| 5 | zweiter Endabschnitt | | |
| 6 | Faserrolle | | |
| 7 | Faserquelle | | |
| 8 | Verstärkungsfaser | | |
| 9 | Faserführung | | |
| 10 | Harzinjektor | | |
| 11 | Faserbündel | | |
| 12 | Formwerkzeug | | |
| 13 | Faserverbundstrang | | |
| 14 | Wickelfaser | | |
| 15 | Wickelfaserrolle | | |
| 16 | Wickelscheibe | | |
| 17 | Wickelscheibe | | |
| 18 | Harzinjektor | | |
| 19 | Formwerkzeug | | |
| 20 | Zieheinheit | | |
| 21 | Schneidevorrichtung | | |
| 22 | Rolle | | |
| 23 | erstes Mantelelement | | |
| 24 | Markierungsmittel | | |
| 25 | zweites Mantelelement | | |
| 26 | erstes Markierungslumen | | |
| 27 | zweites Markierungslumen | | |
| 28 | Außenmantel | | |
| 30 | Führungsdraht | | |

## Patentansprüche

1. Drahtförmiges medizinisches Instrument, umfassend
einen Drahtkern (2) aus einem Faserverbundmaterial, und
mindestens ein zur Markierung bei der Magnetresonanz-Bildgebung dienende flüssiges oder gelförmiges Markierungsmittel (24), wobei das mindestens eine Markierungsmittel (24) in mindestens einem Markierungslumen (26, 27, 33) angeordnet ist,
**dadurch gekennzeichnet, dass**
a) zwischen einem ersten schlauchförmigen Mantelelement (23) und dem Drahtkern (2) das Markierungslumen (26, 27, 33) oder mindestens eines der Markierungslumina (26, 27, 33) mit einem ringförmigen Querschnitt gebildet ist,
und/oder
b) zwischen dem ersten Mantelelement (23) und einem das erste Mantelelement (23) umgebenden schlauchförmigen zweiten Mantelelement (25) das Markierungslumen (26, 27, 33) oder mindestens eines der Markierungslumina (26, 27, 33) mit einem ringförmigen Querschnitt gebildet ist.

2. Medizinisches Instrument nach Anspruch 1, **dadurch gekennzeichnet, dass** der Drahtkern (2) zumindest abschnittsweise schlauchförmig ist und mittels der Schlauchform mindestens ein weiteres Markierungslumen (33) gebildet ist.

3. Medizinisches Instrument nach Anspruch 2, **dadurch gekennzeichnet, dass** das Markierungslumen (33) oder mindestens eines der Markierungslumina (33) des Drahtkerns (2) von einem schlauchförmigen zentralen Lumenelement (31) aus Kunststoff begrenzt wird.

4. Medizinisches Instrument nach einem der vorherigen Ansprüche, **dadurch gekennzeichnet, dass** der Drahtkern (2) mindestens zwei Schichten mit jeweils mindestens einer Wicklung einer oder mehrerer Verstärkungsfasern umfasst, wobei mindestens zwei der Schichten unterschiedliche Wicklungsrichtungen aufweisen.

5. Medizinisches Instrument nach einem der Ansprüche 1 bis 4, **dadurch gekennzeichnet, dass** der Drahtkern (2) an seinem distalen Ende (3) einen ersten Endbereich (4) mit einem gegenüber einem Hauptdurchmesser des Drahtkerns (2) verringerten ersten Enddurchmesser aufweist und das erste Mantelelement (23) den ersten Endbereich (4) umgibt.

6. Medizinisches Instrument nach Anspruch 5, **dadurch gekennzeichnet, dass** der Drahtkern (2) einen in Richtung auf das proximale Ende sich an den ersten Endbereich (4) anschließenden zweiten Endbereich (5) mit einem zweiten Enddurchmesser aufweist, wobei der zweite Enddurchmesser kleiner als der Hauptdurchmesser und größer als der erste Enddurchmesser ist, und
wobei das erste Mantelelement (23) allein den ersten Endbereich (4) und das zweite Mantelelement (25) das erste Mantelelement (23) und den zweiten Endbereich (5) umgibt.

7. Medizinisches Instrument nach einem der Ansprüche 1 bis 6, **dadurch gekennzeichnet, dass** das Markierungsmaterial (24) ein Gel, insbesondere ein Hydrogel, ist.

8. Medizinisches Instrument nach einem der Ansprüche 1 bis 7 **dadurch gekennzeichnet, dass** das Markierungsmaterial (24) ein in der Magnetresonanz-Bildgebung die T1-Relaxationszeit und/oder die T2-Relaxationszeit reduzierendes Agens umfasst.

9. Zur Herstellung eines medizinischen Instruments nach einem der Ansprüche 1 bis 8 geeignetes Verfahren, bei dem
zur Herstellung eines zentralen Drahtkerns (2) mit Harz getränkte erste Verstärkungsfasern (8) mittels Pultrusion durch ein Formwerkzeug (12) gezogen werden und der Drahtkern (2) mit einem ersten Mantelelement (23) umgeben wird.

10. Verfahren nach Anspruch 9, **dadurch gekennzeichnet, dass** zwischen dem Drahtkern (2) und dem ersten Mantelelement eine Schicht aus flüssigem oder gelförmigem Markierungsmittel eingerichtet wird.

11. Verfahren nach Anspruch 9 oder 10, **dadurch gekennzeichnet, dass** der Drahtkern an seinem distalen Ende in einem ersten Endabschnitt (4) gegenüber einem Hauptdurchmesser des Drahtkernes (2) mit einem ersten Enddurchmesser versehen und allein der erste Endabschnitt mit dem ersten Mantelelement (23) umgeben wird.

12. Verfahren nach einem der Ansprüche 9 bis 11, **dadurch gekennzeichnet, dass** das erste Mantelelement (23) mit einem zweiten Mantelelement (25) umgeben wird.

13. Verfahren nach Anspruch 11 und 12, **dadurch gekennzeichnet, dass** der Drahtkern (2) in einem in Richtung auf das proximale Ende hin an den ersten Endabschnitt (4) anschließenden zweiten Endabschnitt (5) mit einem zweiten Enddurchmesser versehen wird, wobei der zweite Enddurchmesser kleiner als der Hauptdurchmesser und größer als der erste Enddurchmesser ist, und sowohl der erste Endabschnitt (4) als auch der zweite Endabschnitt (5) mit dem zweiten Mantelelement (25) umgeben werden.

14. Verfahren nach Anspruch 13, **dadurch gekennzeichnet, dass** zwischen dem zweiten Mantelelement (25) und dem vom zweiten Mantelelement (25) umgebenen zweiten Endabschnitt (5) sowie dem ersten Mantelelement (23) eine Schicht aus flüssigem oder gelförmigem Markierungsmittel (24) eingerichtet wird.

15. Verfahren nach einem der Ansprüche 9 bis 14, **dadurch gekennzeichnet, dass** zur Bildung des zentralen Drahtkerns (2) ein schlauchförmiges, mindestens ein zentrales Markierungslumen (33) aufweisendes Lumenelement (31) aus Kunststoff zusammen mit den ersten Verstärkungsfasern (8) in Harz getränkt mittels Pultrusion durch das Formwerkzeug (12) gezogen wird und das mindestens eine zentrale Markierungslumen (33) mit einem flüssigen oder gelfömigen Markierungsmittel (24) gefüllt wird.

16. Verfahren nach einem der Ansprüche 9 bis 15, **dadurch gekennzeichnet, dass** der zentrale Drahtkern (2) während des Pultrusionsvorganges im Anschluss an das Formwerkzeug (12) mit mindestens zwei Wickelschichten versehen wird, welche zweite Verstärkungsfasern (14) aufweisen, wobei mindestens zwei Wickelschichten unterschiedliche Wicklungsrichtungen aufweisen.

## Claims

1. Wire-shaped medical instrument, comprising
a wire core (2) made of a fibre composite material, and
at least one liquid or gel-like marking agent (24) that serves for marking in magnetic resonance imaging, wherein the at least one marking agent (24) is arranged in at least one marking lumen (26, 27, 33),
**characterized in that**
a) between a first tubular jacket element (23) and the wire core (2), the marking lumen (26, 27, 33) or at least one of the marking lumens (26, 27, 33) is formed with an annular cross section,
and/or
b) between the first jacket element (23) and a tubular second jacket element (25) surrounding the first jacket element (23), the marking lumen (26, 27, 33) or at least one of the marking lumens (26, 27, 33) is formed with an annular cross section.

2. Medical instrument according to Claim 1, **characterized in that** the wire core (2) is tubular at least in sections, and at least one further marking lumen (33) is formed by means of the tube shape.

3. Medical instrument according to Claim 2, **characterized in that** the marking lumen (33) or at least one of the marking lumens (33) of the wire core (2) is delimited by a tubular central lumen element (31) made of plastic.

4. Medical instrument according to one of the preceding claims, **characterized in that** the wire core (2) comprises at least two layers, each with at least one winding of one or more reinforcing fibres, wherein at least two of the layers have different winding directions.

5. Medical instrument according to one of Claims 1 to 4, **characterized in that** the wire core (2) has, at its distal end (3), a first end region (4) with a first end diameter reduced in relation to a main diameter of the wire core (2), and the first jacket element (23) surrounds the first end region (4) .

6. Medical instrument according to Claim 5, **characterized in that** the wire core (2) has, adjoining the first end region (4) in the direction towards the proximal end, a second end region (5) with a second end diameter, wherein the second end diameter is smaller than the main diameter and greater than the first end diameter, and
wherein the first jacket element (23) alone surrounds the first end region (4), and the second jacket element (25) surrounds the first jacket element (23) and the second end region (5).

7. Medical instrument according to one of Claims 1 to 6, **characterized in that** the marking material (24) is a gel, in particular a hydrogel.

8. Medical instrument according to one of Claims 1 to 7, **characterized in that** the marking material (24) comprises an agent reducing the T1 relaxation time and/or the T2 relaxation time in magnetic resonance imaging.

9. Method suitable for producing a medical instrument according to one of Claims 1 to 8, in which method, in order to produce a central wire core (2), resin-impregnated first reinforcing fibres (8) are drawn by means of pultrusion through a die (12), and the wire core (2) is surrounded by a first jacket element (23).

10. Method according to Claim 9, **characterized in that** a layer of liquid or gel-like marking agent is arranged between the wire core (2) and the first jacket element.

11. Method according to Claim 9 or 10, **characterized in that** the wire core is provided at its distal end, in a first end portion (4) relative to a main diameter of the wire core (2), with a first end diameter, and only the first end portion is surrounded by the first jacket element (23).

12. Method according to one of Claims 9 to 11, **characterized in that** the first jacket element (23) is surrounded by a second jacket element (25) .

13. Method according to Claims 11 and 12, **characterized in that** the wire core (2) is provided with a second end diameter in a second end portion (5) adjoining the first end portion (4) in the direction towards the proximal end, wherein the second end diameter is smaller than the main diameter and greater than the first end diameter, and both the first end portion (4) and the second end portion (5) are surrounded by the second jacket element (25).

14. Method according to Claim 13, **characterized in that** a layer of liquid or gel-like marking agent (24) is arranged between the second jacket element (25) and the second end portion (5) surrounded by the second jacket element (25) and also the first jacket element (23).

15. Method according to one of Claims 9 to 14, **characterized in that**, in order to form the central wire core (2), a tubular lumen element (31) made of plastic and having at least one central marking lumen (33) is drawn by means of pultrusion through the die (12) together with the first reinforcing fibres (8) soaked in resin, and the at least one central marking lumen (33) is filled with a liquid or gel-like marking agent (24) .

16. Method according to one of Claims 9 to 15, **characterized in that**, during the pultrusion process, the central wire core (2) is provided, downstream from the die (12), with at least two winding layers which have second reinforcing fibres (14), wherein at least two winding layers have different winding directions.

## Revendications

1. Instrument médical en forme de fil, comprenant une âme de fil (2) en un matériau composite à base de fibres et
au moins un moyen de marquage (24) liquide ou sous forme de gel servant au marquage lors de l'imagerie par résonance magnétique, l'au moins un moyen de marquage (24) étant disposé dans au moins une lumière de marquage (26, 27, 33),
**caractérisé en ce que**
a) la lumière de marquage (26, 27, 33) ou au moins une des lumières de marquage (26, 27, 33) est formée avec une section transversale de forme annulaire entre un premier élément d'enveloppe (23) tubulaire et l'âme de fil (2),
et/ou
b) la lumière de marquage (26, 27, 33) ou au moins une des lumières de marquage (26, 27, 33) est formée avec une section transversale de forme annulaire entre le premier élément d'enveloppe (23) et un deuxième élément d'enveloppe (25) tubulaire qui entoure le premier élément d'enveloppe (23).

2. Instrument médical selon la revendication 1, **caractérisé en ce que** l'âme de fil (2) est de forme tubulaire au moins dans certaines portions et au moins une lumière de marquage supplémentaire (33) est formée au moyen de la forme tubulaire.

3. Instrument médical selon la revendication 2, **caractérisé en ce que** la lumière de marquage (33) ou au moins l'une des lumières de marquage (33) de l'âme de fil (2) est délimitée par un élément de lumière central (31) de forme tubulaire en matière plastique.

4. Instrument médical selon l'une des revendications précédentes, **caractérisé en ce que** l'âme de fil (2) comprend au moins deux couches ayant respectivement au moins un enroulement d'une ou plusieurs fibres de renforcement, au moins deux des couches possédant des sens d'enroulement différents.

5. Instrument médical selon l'une des revendications 1 à 4, **caractérisé en ce que** l'âme de fil (2) possède à son extrémité distale (3) une première zone d'extrémité (4) ayant un premier diamètre d'extrémité réduit par rapport à un diamètre principal de l'âme de fil (2) et le premier élément d'enveloppe (23) entoure la première zone d'extrémité (4).

6. Instrument médical selon la revendication 5, **caractérisé en ce que** l'âme de fil (2) possède une deuxième zone d'extrémité (5) ayant un deuxième diamètre d'extrémité qui se rattache à la première zone d'extrémité (4) dans la direction de l'extrémité proximale, le deuxième diamètre d'extrémité étant inférieur au diamètre principal et supérieur au premier diamètre d'extrémité, et
le premier élément d'enveloppe (23) entourant seul la première zone d'extrémité (4) et le deuxième élément d'enveloppe (25) entourant le premier élément d'enveloppe (23) et la deuxième zone d'extrémité (5).

7. Instrument médical selon l'une des revendications 1 à 6, **caractérisé en ce que** le matériau de marquage (24) est un gel, notamment un hydrogel.

8. Instrument médical selon l'une des revendications 1 à 7 **caractérisé en ce que** le matériau de marquage (24) comprend un agent qui réduit le temps de relaxation T1 et/ou le temps de relaxation T2 dans l'imagerie par résonance magnétique.

9. Procédé adapté à la fabrication d'un instrument médical selon l'une des revendications 1 à 8, selon lequel, en vue de la fabrication d'une âme de fil centrale (2), des premières fibres de renforcement (8) imprégnées de résine sont tirées par pultrusion à travers un outil de formage (12) et l'âme de fil (2) est entourée d'un premier élément d'enveloppe (23).

10. Procédé selon la revendication 9, **caractérisé en ce qu'**une couche constituée d'un moyen de marquage liquide ou sous forme de gel est mise en place entre l'âme de fil (2) et le premier élément d'enveloppe.

11. Procédé selon la revendication 9 ou 10, **caractérisé en ce que** l'âme de fil est pourvue, au niveau de son extrémité distale dans une première portion d'extrémité (4), d'un premier diamètre d'extrémité par rapport à un diamètre principal de l'âme de fil (2) et seule la première portion d'extrémité est entourée par le premier élément d'enveloppe (23).

12. Procédé selon l'une des revendications 9 à 11, **caractérisé en ce que** le premier élément d'enveloppe (23) est entouré par un deuxième élément d'enveloppe (25) .

13. Procédé selon les revendications 11 et 12, **caractérisé en ce que** l'âme de fil (2), dans une deuxième portion d'extrémité (5) qui se rattache à la première portion d'extrémité (4) en direction de l'extrémité proximale, est pourvue d'un deuxième diamètre d'extrémité, le deuxième diamètre d'extrémité étant inférieur au diamètre principal et supérieur au premier diamètre d'extrémité, et la première portion d'extrémité (4) ainsi que la deuxième portion d'extrémité (5) sont toutes deux entourées par le deuxième élément d'enveloppe (25).

14. Procédé selon la revendication 13, **caractérisé en ce qu'**entre le deuxième élément d'enveloppe (25) et la deuxième portion d'extrémité (5) entourée par le deuxième élément d'enveloppe (25) ainsi que le premier élément d'enveloppe (23) est mise en place une couche constituée d'un moyen de marquage (24) liquide ou sous forme de gel.

15. Procédé selon l'une des revendications 9 à 14, **caractérisé en ce que** pour former l'âme de fil (2) centrale, un élément formant lumière (31) en matière plastique de forme tubulaire, possédant au moins une lumière de marquage centrale (33), imprégné de résine conjointement avec les premières fibres de renforcement (8), est tiré par pultrusion à travers l'outil de formage (12) et l'au moins une lumière de marquage centrale (33) est remplie d'un moyen de marquage (24) liquide ou sous forme de gel.

16. Procédé selon l'une des revendications 9 à 15, **caractérisé en ce que** l'âme de fil (2) centrale, pendant l'opération de pultrusion, est pourvue d'au moins deux couches enroulées à la suite de l'outil de formage (12), lesquelles possèdent des deuxièmes fibres de renforcement (14), au moins deux couches enroulées possédant des sens d'enroulement différents.
